# EUROPÄISCHE PATENTSCHRIFT

(11) **EP 0 493 721 B1**
(45) Veröffentlichungstag und Bekanntmachung des Hinweises auf die Patenterteilung: **14.05.1997**
(21) Anmeldenummer: 91121314.8
(22) Anmeldetag: 12.12.1991
(51) Int. Cl.: C07D 207/452, C07D 209/48, C07D 231/56, C07D 513/04, A01N 43/36, A01N 43/38, A01N 43/56, A01N 43/90

(54) **Styrolderivate**
Styrene derivatives
Dérivés de styrène

(30) Priorität: 29.12.1990 DE 4042194
(43) Veröffentlichungstag der Anmeldung: 08.07.1992
(73) Patentinhaber: BASF Aktiengesellschaft, 67063 Ludwigshafen (DE)
(72) Erfinder: Schaefer, Bernd, Dr., W-6749 Dierbach (DE); Rueb, Lothar, Dr., W-6720 Speyer (DE); Schaefer, Peter, Dr., W-6702 Bad Duerkheim (DE); Westphalen, Karl-Otto, Dr., W-6720 Speyer (DE); Gerber, Matthias, Dr., W-6704 Mutterstadt (DE); Walter, Helmut, Dr., W-6719 Obrigheim (DE)

(56) Entgegenhaltungen:
- DE-A- 3 603 789
- DE-A- 3 714 373
- DE-A- 3 724 399
- DE-A- 3 901 705
- DE-A- 3 917 676

## Beschreibung

Die vorliegende Erfindung betrifft neue Styrolderivate der allgemeinen Formel I 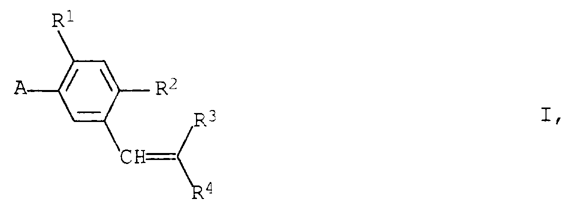 in der die Substituenten folgende Bedeutung haben:
- R¹: Wasserstoff oder Halogen;
- R²: Halogen;
- R³: Wasserstoff, Halogen oder C₁-C₄-Alkyl;
- R⁴: Cyano oder C₁-C₆-Alkylcarbonyl;
- A: einen heterocyclischen Rest, ausgewählt aus der Gruppe A₁ bis A₄ 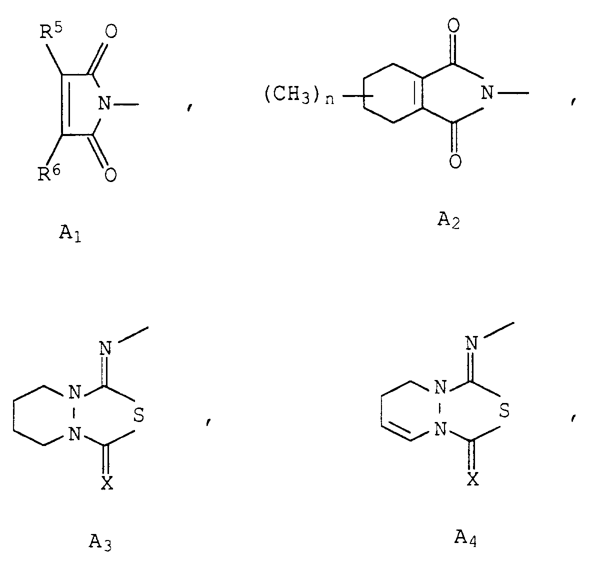 wobei R⁵ und R⁶ Wasserstoff, Methyl oder Ethyl bedeuten, n für 0 oder 1 und X für Sauerstoff oder Schwefel stehen;
mit der Maßgabe, daß R² Chlor und R⁴ C₁-C₆-Alkylcarbonyl bedeuten, wenn A für den Rest A₂ steht;
sowie deren landwirtschaftlich brauchbaren Salze, wobei die Formel I sämtliche isomere Formen dieser Verbindungen umfaßt.

Des weiteren betrifft die Erfindung Verfahren zur Herstellung dieser Verbindungen sowie herbizide Mittel und Verfahren zur Entblätterung (Desikkation) von Baumwolle.

Aus der japanischen Offenlegungsschrift JP-A Kokai 27962/1986 und der DE-A 37 24 399 sind Zimtsäureester der Struktur I' bekannt 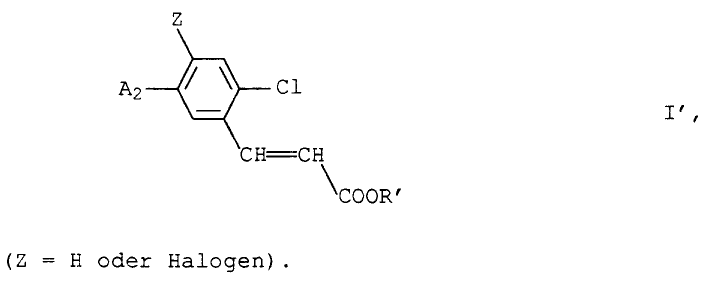 Des weiteren werden in der DE-A 37 14 373 N-Phenyl-3,4,5,6-tetrahydrophthalimide als Herbizide beschrieben, die sich von den vorliegenden Verbindungen I mit A = A₂ dadurch unterscheiden, daß der Phenylring anstelle des Rests -CH=C(R³,R⁴) eine mono- oder disubstituierte Methylgruppe trägt.

Außerdem fallen diejenigen Verbindungen I mit A = A₂, n = 0, R¹ = Wasserstoff und R³ = Wasserstoff, Chlor, Brom oder C₁-C₄-Alkyl unter die allgemeine Lehre der DE-A 36 03 789, diejenigen mit A = A₂, n = 1 und R¹ = H oder Fluor unter die allgemeine Lehre der DE-A 39 17 676.

Da die Verbindungen des o.g. Standes der Technik entweder nicht ausreichende Selektivität aufweisen oder hinsichtlich der benötigten Aufwandmengen zu wünschen übrig lassen, lag der Erfindung die Aufgabe zugrunde, Verbindungen zur Verfügung zu stellen, die herbizid besonders gut wirksam sind. Weiterhin sollten die Verbindungen als Bioregulatoren, insbesondere zur Entblätterung von Baumwolle, verwendbar sein.

Es wurde nun gefunden, daß die eingangs definierten Styrolderivate I auch bei geringen Aufwandmengen eine gute herbizide Wirksamkeit besitzen und sehr gut zur Entblätterung von Baumwolle geeignet sind.

Man erhält die Verbindungen der Formel I, indem man einen geeignet substituierten Benzaldehyd der Formel II mit einem Phosphoran der Formel III in an sich bekannter Weise, z.B. analog den in Synthesis 10 (1984) 862 beschriebenen Bedingungen, in einem Lösungsmittel bei Temperaturen zwischen 0°C und dem Siedepunkt des Lösungsmittels umsetzt:

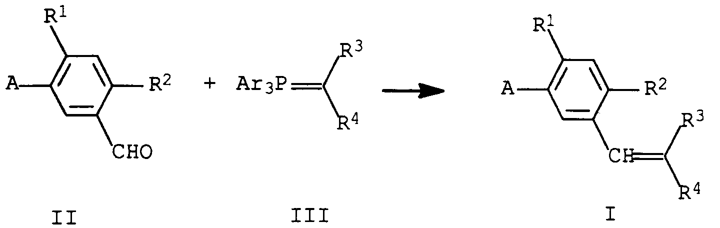

Ar in Formel III bedeutet einen unsubstituierten oder substituierten Arylrest, wobei im allgemeinen der Phenylrest bevorzugt wird.

Die zur Herstellung der Styrolderivate I benötigten Phosphorane III, welche auch als Phosphor-Ylide bezeichnet werden, sind nach literaturbekannten Methoden (z.B. Houben-Weyl, Methoden der Organischen Chemie, Bd. E1, S. 636-639, Georg-Thieme Verlag, Stuttgart 1982 oder Chem. Ber. 95, 1962, Seite 3993) erhältlich.

Die Gruppen Ar dieser Phosphorane können unsubstituierte oder substituierte Phenylreste sein. Die Zahl der Substituenten pro Phenylrest sowie das Substitutionsmuster der Phenylreste sind in der Regel für das Gelingen des Verfahrens nicht kritisch, im allgemeinen trägt ein Phenylrest aber nicht mehr als 3 Substituenten. Als Substituenten der Phenylreste sind solche bevorzugt, welche sich unter den Bedingungen des Verfahrens inert verhalten, z.B. die Halogenide Fluor, Chlor und Brom, Alkylgruppen, bevorzugt C₁- bis C₄-Alkylgruppen, insbesondere die Methylgruppe, oder C₁- bis C₄-Alkoxygruppen, insbesondere die Methoxygruppe. Allerdings werden in der Regel Triarylphosphorane III mit unsubstituiertem Phenylrest bevorzugt.

Die Umsetzung der Ausgangsverbindungen II und III wird im allgemeinen vorteilhaft in Gegenwart eines Lösungsmittels durchgeführt. Als Lösungsmittel können alle üblicherweise bei der Durchführung von Wittig-Reaktionen verwendeten Lösungsmittel Anwendung finden, beispielsweise halogenierte Lösungsmittel wie Chloroform, oder Ether wie Tetrahydrofuran, Dioxan und Ethylenglykoldimethylether. Bevorzugte Lösungsmittel sind Alkohole, insbesondere C₁- bis C₄-Alkohole wie Methanol, Ethanol, Propanol, Isopropanol, Butanol, Isobutanol und tert.-Butanol. Die Lösungsmittel können auch in Form von Lösungsmittelgemischen eingesetzt werden, in der Regel werden jedoch bevorzugt die reinen Lösungsmittel verwendet.

Im allgemeinen wird die Reaktion bei Temperaturen zwischen 0°C und 100°C ausgeführt. Es versteht sich jedoch von selbst, daß die optimale Reaktionstemperatur von den jeweils umzusetzenden Ausgangsverbindungen II und III sowie dem verwendeten Lösungsmittel abhängig ist.

Die Ausgangsverbindungen II und III können in stöchiometrischen Mengen miteinander umgesetzt werden. Es kann sich jedoch als vorteilhaft erweisen, wenn einer der beiden Reaktanden, II oder III, in einem 0,05-5fachen molaren Überschuß in die Reaktion eingesetzt wird.

Der Verlauf der Wittig-Reaktion kann mittels üblicher analytischer Methoden, wie Dünnschichtchromatographie und Hochdruckflüssigkeitschromatographie, verfolgt werden. Nach beendeter Reaktion kann das Produkt I nach herkömmlichen Verfahren wie Filtration, Zentrifugation oder durch Zugabe von Wasser mit anschließender Extraktion isoliert werden. Gewünschtenfalls können die so erhaltenen Styrolderivate I beispielsweise durch Umkristallisation oder mittels chromatographischer Methoden weiter gereinigt werden.

Nach vorstehendem Verfahren werden die Styrolderivate I hinsichtlich der Alkenylseitenkette im allgemeinen als cis-trans-Isomerengemische erhalten, wobei in der Regel das trans-Isomere überwiegt.

Das Verfahren zur Herstellung der Styrolderivate der Formel I beinhaltet weiterhin die Möglichkeit, die Reihenfolge in der Synthese der Benzaldehyde der allgemeinen Formel II und die Wittig-Olefinierung zu vertauschen.

Es besteht insbesondere die Möglichkeit geeignete Vorstufen der Benzaldehyde II mit den Phosphoranen III umzusetzen, und erst dann die entsprechenden Substituenten im Benzaldehydteil zu der Struktur der allgemeinen Formel I zu ergänzen (wie weiter unten beschrieben).

Die als Ausgangsmaterial verwendeten Benzaldehyde der allgemeinen Formel II sind nach den Methoden der DE-A 38 15 042 (≙ EP-A 340 708) auf einfache Weise zugänglich oder können beispielsweise wie nachfolgend beschrieben hergestellt werden.

Man erhält die Benzaldehyde der Formel IIc, d wenn man ein Anilinderivat der Formel IV entsprechend den für IX geschilderten Bedingungen zuerst mit Thiophosgen in das entsprechende Isothiocyanat V überführt, V an ein Piperazin VI addiert, das so erhaltene Thioharnstoffderivat VII unter saurer Spaltung der Acetalgruppe zur Aldehydfunktion mit einem Phosgenierungsmittel oder zum Aldehyd IIc, d cyclisiert:

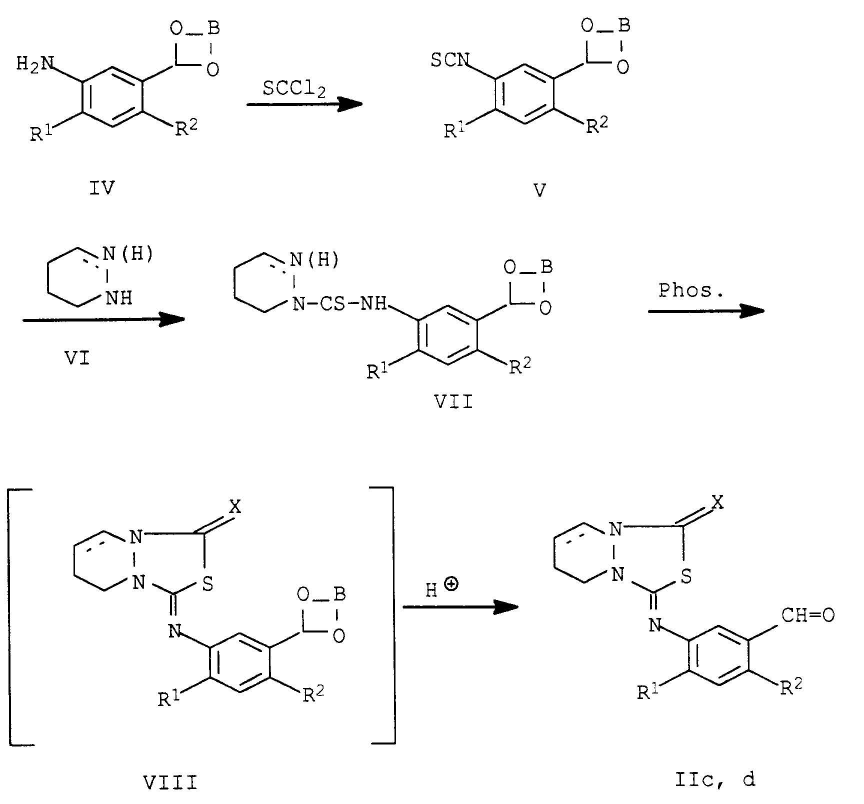

In den Formeln IV, V, VII, VIII bedeutet B eine Ethylen- oder Propyleneinheit, welche ein bis drei Alkylgruppen wie Methyl, Ethyl, Propyl und 1-Methylethyl, vorzugsweise Methyl, tragen kann. Die gepunktete Linie in den Formeln VI, VII, VIII und IIc, d steht für eine ggf. vorhandene π-Bindung.

Die Umsetzungen von IV zu V, von V zu VII und von VII zu VIII verlaufen unter den nachstehend geschilderten Bedingungen analog zur zweiten Synthesevariante von Ic, d.

Die Acetalgruppe in Verbindung VIII wird unter sauren Bedingungen beispielsweise in Gegenwart von Mineralsäuren wie Salzsäure und Schwefelsäure oder organischen Säuren wie p-Toluolsulfonsäure in die Aldehydfunktion übergeführt.

Man erhält die Verbindungen I mit den Resten A₁ und A₂ aber beispielsweise auch dadurch, daß man ein Anilin der allgemeinen Formel IX mit Anhydriden der allgemeinen Formel Xa oder Xb kondensiert:

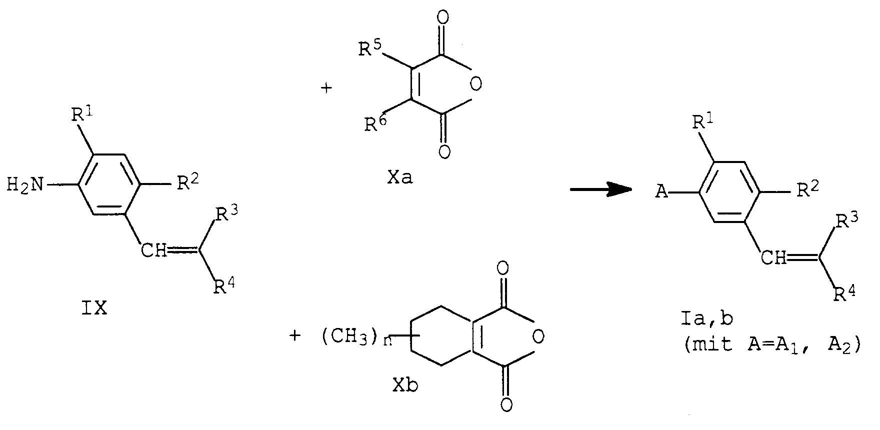

Für die Kondensation dienen inerte organische Lösungsmittel wie niedere Alkansäuren wie Essigsäure, Propionsäure und Isobuttersäure, sowie die Ester dieser Säuren wie Essigsäureethylester, höhersiedende Kohlenwasserstoffe wie Toluol und Xylol und/oder Dimethylformamid. Die Umsetzung erfolgt in der Regel bei Temperaturen zwischen 25°C und dem Siedepunkt der jeweiligen Reaktionsmischung, vorzugsweise zwischen 50 und 140°C. Bei Verwendung eines aprotischen Lösungsmittels empfiehlt es sich, das Wasser fortlaufend zu entfernen.

Die Aniline IX erhält man beispielsweise durch Reduktion aus den entsprechenden Nitroderivaten analog bekannter Methoden (DE-A 37 24 399, DE-A 36 03 789):

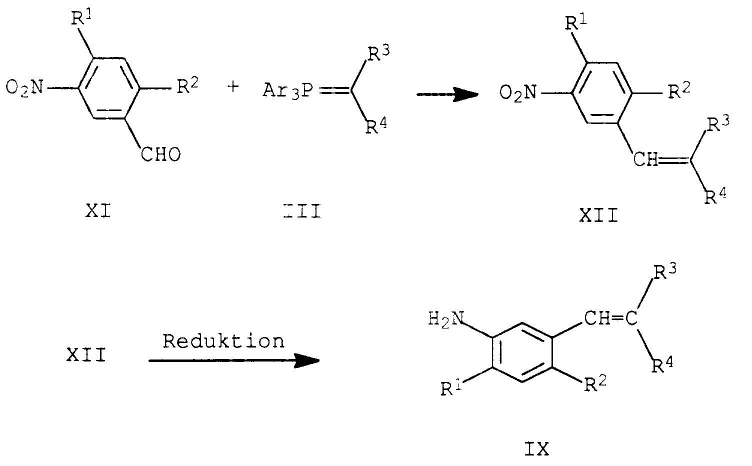

Die Umsetzung der Nitrobenzaldehyde XI mit den Phosphoranen III erfolgt analog zu den oben beschriebenen Methoden.

In der zweiten Stufe der Reaktionsfolge wird ein Nitrostyrol XII in an sich bekannter Weise mit anorganischen Verbindungen wie Zinn-II-salzen oder Eisen oder Zinn oder durch katalytische Hydrierung an Metallkontakten wie Raney-Nickel, Palladium und Platin in einem inerten organischen Lösungsmittel zum Anilinderivat IX reduziert.

Geeignete Lösungsmittel für die Reaktion mit anorganischen Verbindungen sind beispielsweise Alkohole wie Methanol, Ethanol und Isopropanol und niedere Alkansäuren wie Ameisensäure, Essigsäure und Propionsäure sowie entsprechende Gemische. Die Reaktionstemperaturen liegen bei 25°C bis 150°C, vorzugsweise bei 25°C bis 100°C.

Sofern man die Reduktion an Metallkontakten mit Wasserstoff durchführt, eignen sich Lösungsmittel wie Methanol oder andere Alkohole, Dimethylformamid, Propionsäure, Tetrahydrofuran und Eisessig und entsprechende Gemische bei einem Wasserstoffdruck von 1 bis 150 bar, vorzugsweise 1 bis 50 bar und Temperaturen von 25°C bis 100°C, vorzugsweise 25 bis 70°C.

Man erhält die Verbindungen Ic, d beispielsweise aber auch dadurch, daß man ein Derivat der allgemeinen Formel IX in an sich bekannter Weise (Houben-Weyl, Bd. IX, S. 867 f. (1955)) in einem inerten organischen Lösungsmittel mit Thiophosgen in das entsprechende Isothiocyanat XIII überführt, XIII anschließend in einem aprotisch polaren Lösungsmittel an ein Tetrahydro- oder Perhydrodiazinderivat VI addiert und den so erhaltenen Thioharnstoff XIV mit einem Phosgenierungsmittel oder Thiophosgenierungsmittel (Phos.) zu Ic, d cyclisiert:

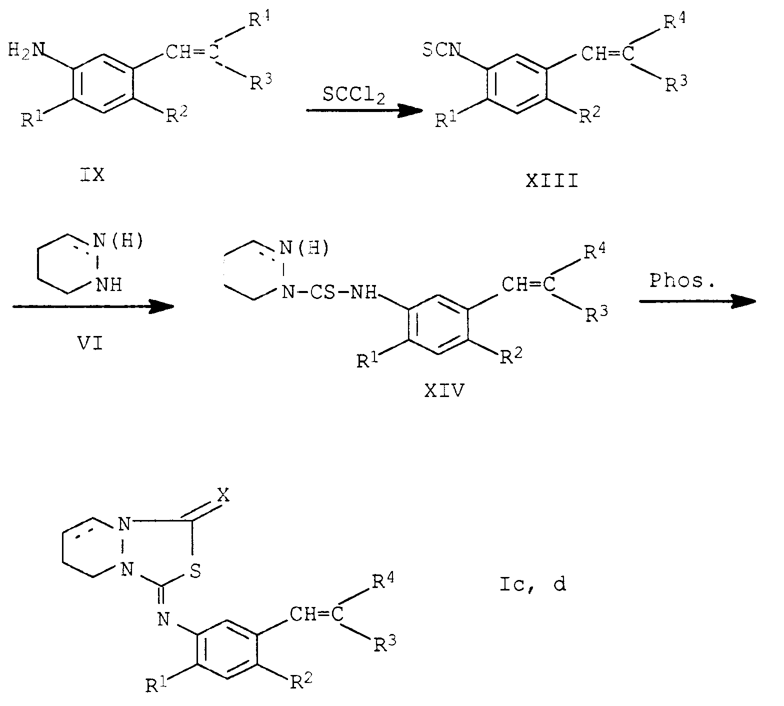

Die Umsetzung des Derivats IX mit Thiophosgen erfolgt in der Regel bei Temperaturen von (-50)°C bis 100°C, vorzugsweise 0°C bis 50°C.

Diese Umsetzung kann sowohl in einem Zweiphasen-Lösungsmittelsystem wie Methylenchlorid/Wasser als auch in Gegenwart einer Base in einem aprotisch polaren organischen Lösungsmittel durchgeführt werden.

Im letztgenannten Fall arbeitet man bevorzugt in Toluol in Gegenwart einer organischen Base, vorzugsweise eines tertiären Amins wie Triethylamin.

Die Umsetzung der Isothiocyanate XIII mit den Piperazinen VI erfolgt bevorzugt in aprotisch polaren Lösungsmitteln wie vorzugsweise Ethern, insbesondere Tetrahydrofuran bei Temperaturen von (-50)°C bis 100°C, vorzugsweise 0°C bis 50°C.

Die anschließende Cyclisierung des Thioharnstoffs XIV mit einem Phosgenierungsmittel oder Thiophosgenierungsmittel geschieht in der Regel bei Temperaturen von 0°C bis 100°C, vorzugsweise 20°C bis 70°C in einem aprotisch polaren Lösungsmittel in Gegenwart einer Base.

Als Phosgenierungsmittel und Thiophosgenierungsmittel eignen sich insbesondere Phosgen, Thiophosgen und Chlorameisensäuretrichlormethylester.

Als Lösungsmittel dienen vorzugsweise Ether, Halogenkohlenwasserstoffe und Kohlenwasserstoffe, insbesondere Halogenkohlenwasserstoffe wie Methylenchlorid.

Geeignete Basen Sind tertiäre Amine wie insbesondere Pyridin.

Die Styrolderivate I können bei der Herstellung als Isomerengemische anfallen, wobei Enantiomeren- oder Diastereomerengemische und insbesondere E-/Z-Isomerengemische möglich sind. Die Isomerengemische können gewünschtenfalls nach den hierfür üblichen Methoden, z.B. durch Chromatographie oder durch Kristallisation, getrennt werden.

Im Hinblick auf die bestimmungsgemäße Verwendung der Verbindungen I als Herbizide kommen als Substituenten vorzugsweise folgende Reste in Betracht:
- R¹: Wasserstoff oder Halogen, wie Fluor, Chlor und Brom, insbesondere Wasserstoff oder Fluor;
- R²: Halogen wie Fluor, Chlor und Brom, insbesondere Chlor;
- R³: Wasserstoff; ein Halogenatom wie unter R² genannt, aber auch Jod, insbesondere Chlor und Brom; eine verzweigte oder unverzweigte C₁-C₄-Alkylgruppe wie Methyl, Ethyl, n-Propyl, Isopropyl, n-Butyl, 1-Methylpropyl, 2-Methylpropyl und 1,1-Dimethylethyl, insbesondere Methyl und Ethyl;
- R⁴: Cyano; C₁-C₆-Alkylcarbonyl, wobei als Alkylreste Methyl, Ethyl, n-Propyl, Isopropyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl und 1-Ethyl-2-methylpropyl, insbesondere Methyl, Ethyl, n-Propyl und Isopropyl in Betracht kommen;
- R⁵ und R⁶: Wasserstoff, Methyl und Ethyl.

Die Styrolderivate I können in Form ihrer landwirtschaftlich brauchbaren Salze vorliegen. Allgemein eignen sich die Salze von solchen Säuren, welche die herbizide Wirkung von I nicht beeinträchtigen, beispielsweise Alkalimetallsalze, insbesondere das Natrium- oder Kaliumsalz, Erdalkalisalze, insbesondere das Calcium-, Magnesium- oder Bariumsalz, Mangan-, Kupfer-, Zink- oder Eisensalz sowie Ammonium-, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumslaze.

Beispielsweise seien die in den nachstehenden Tabellen 1 bis 5 aufgeführten Verbindungen I genannt.

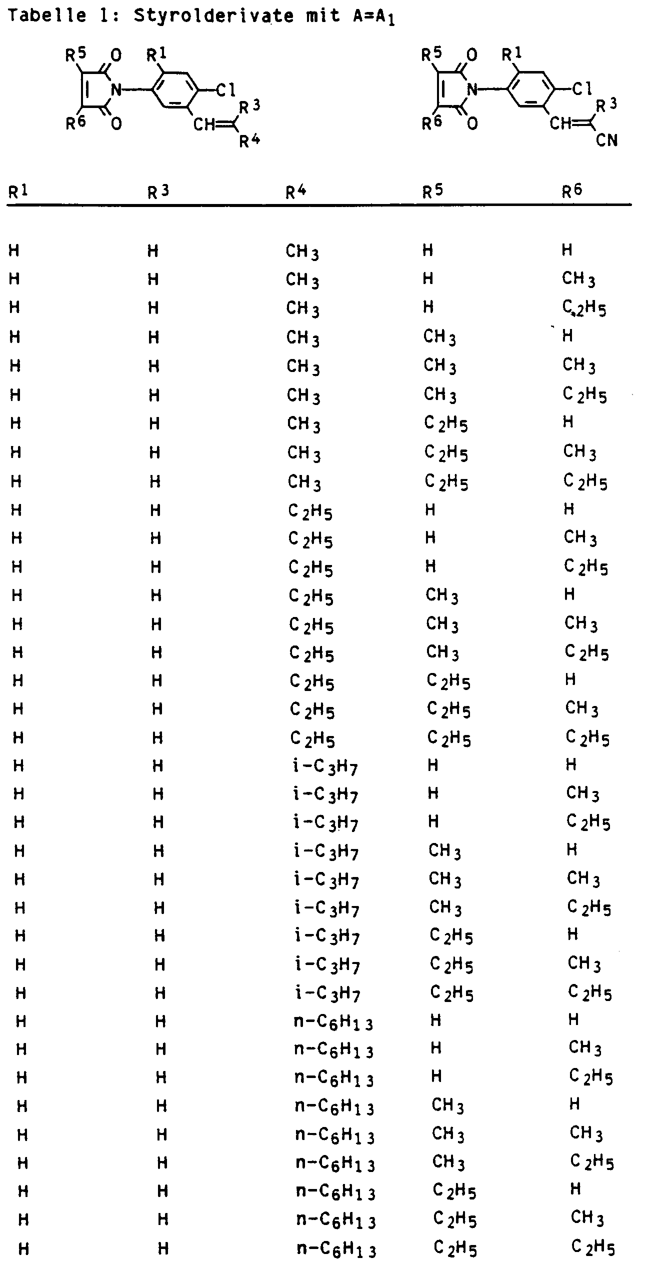

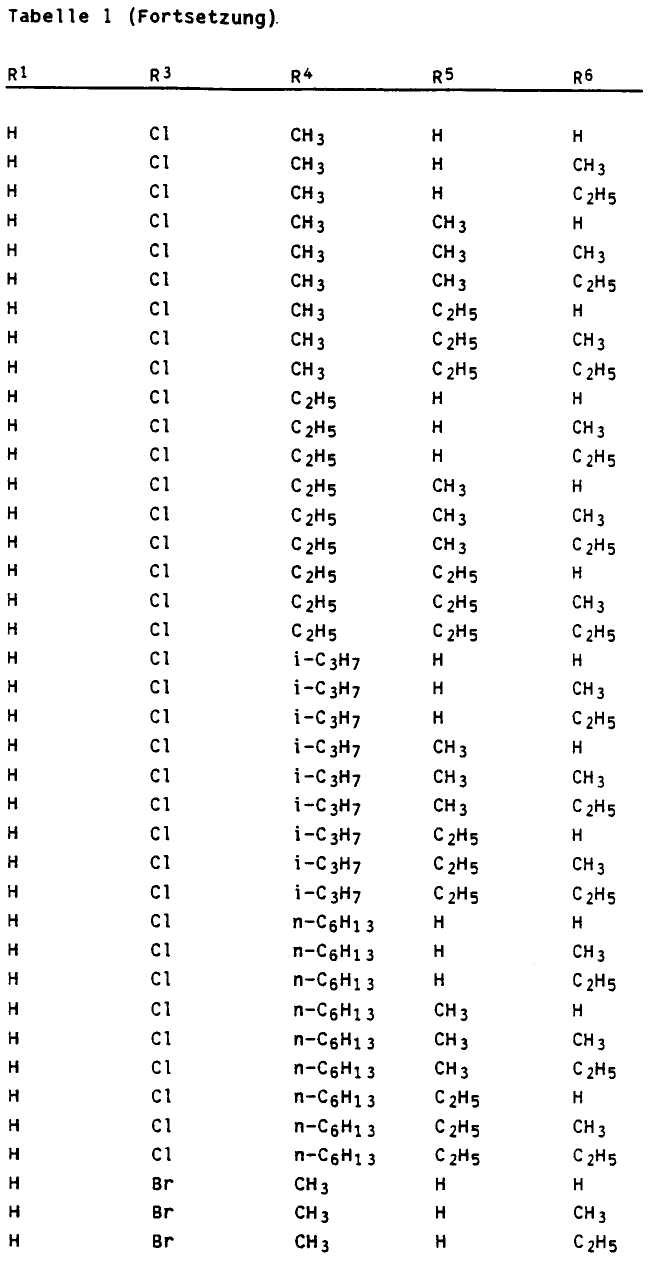

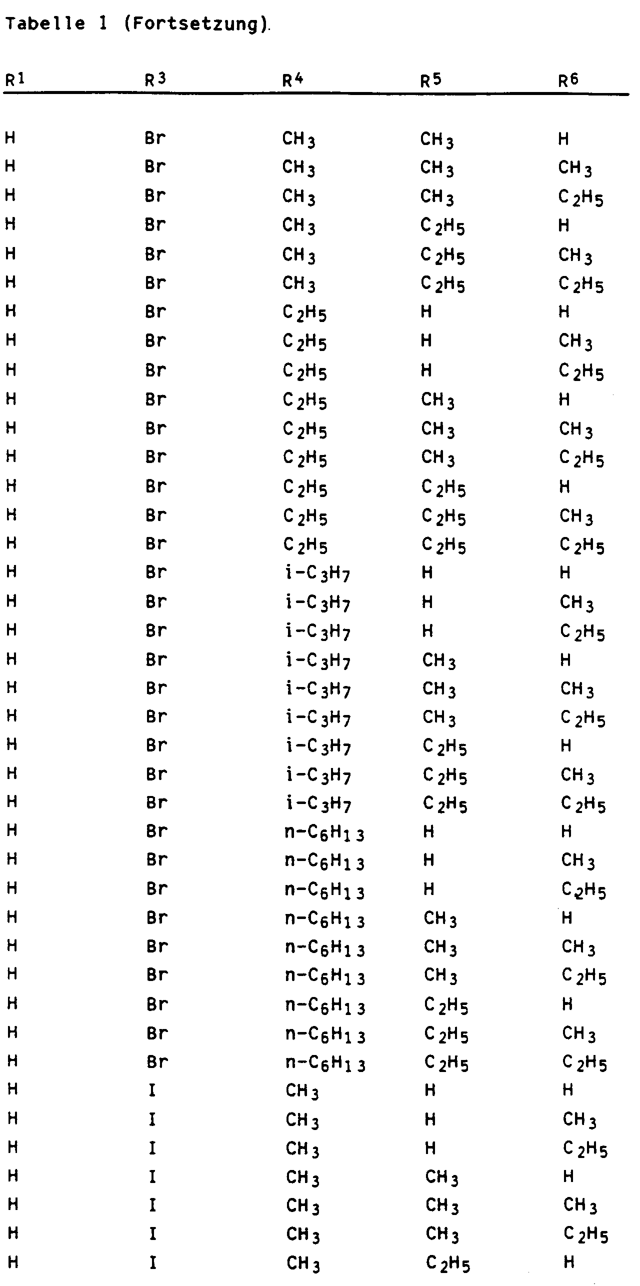

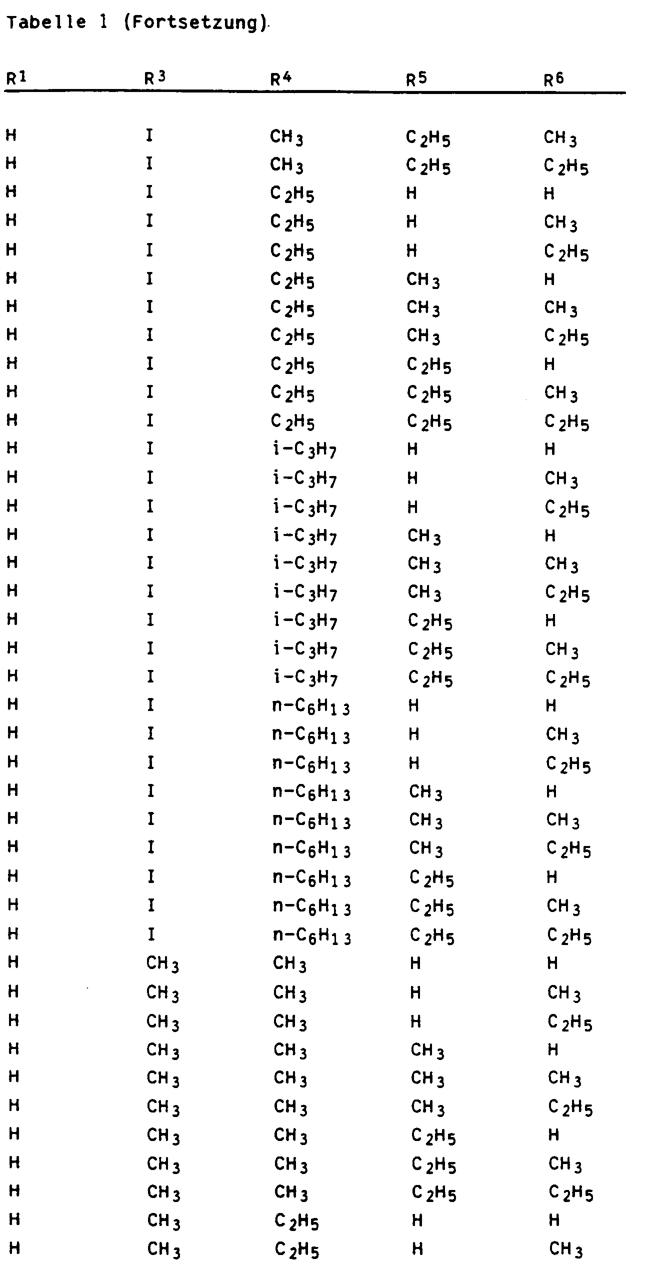

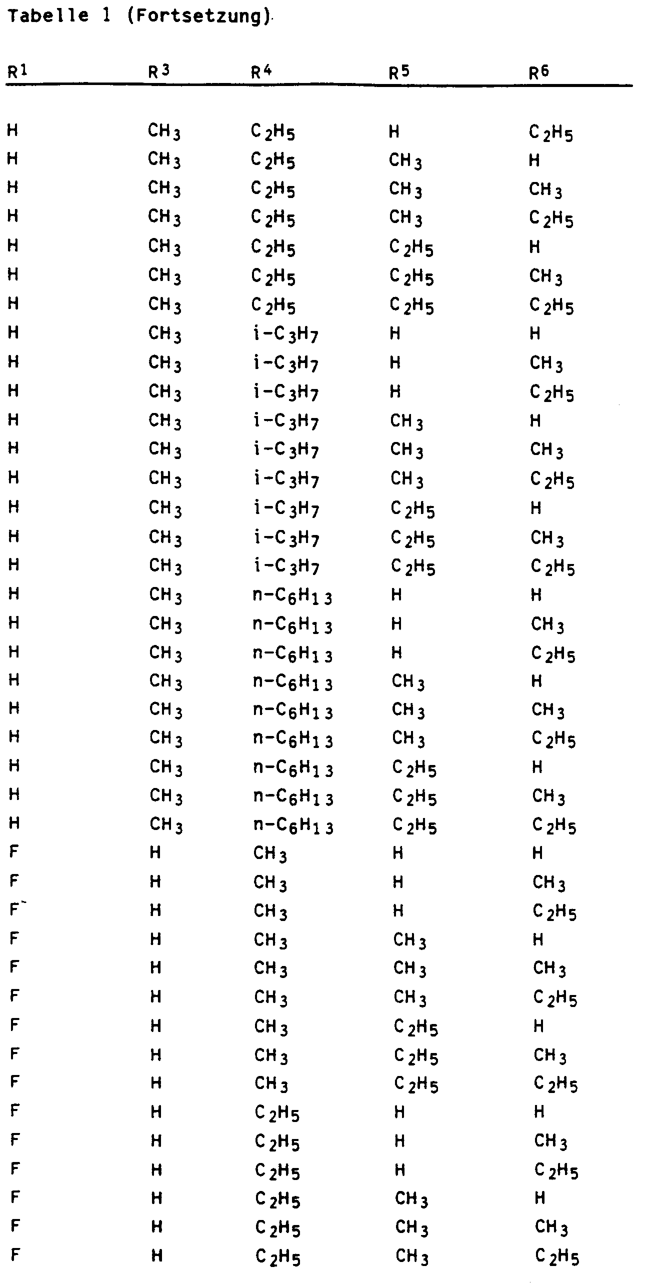

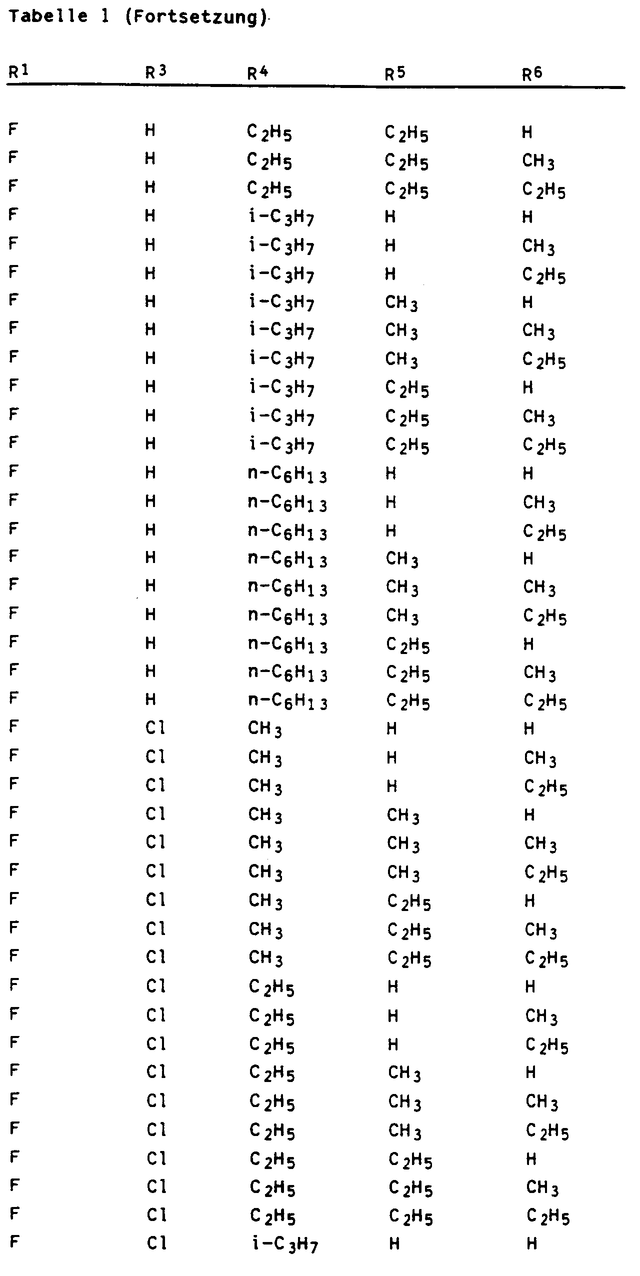

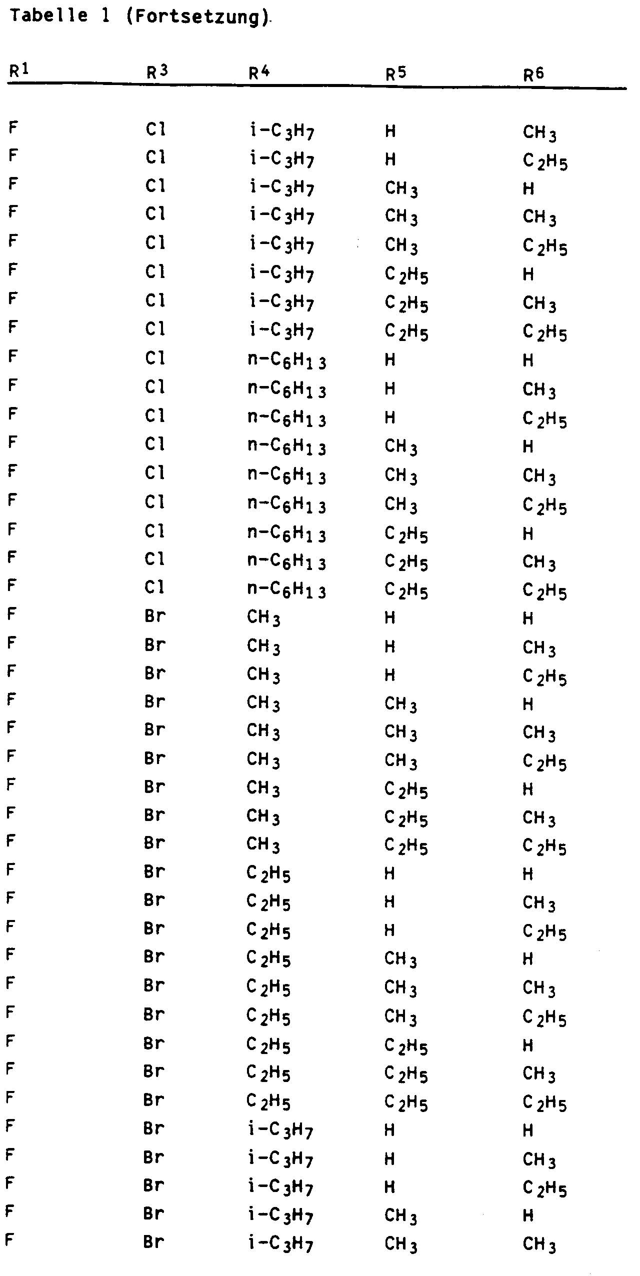

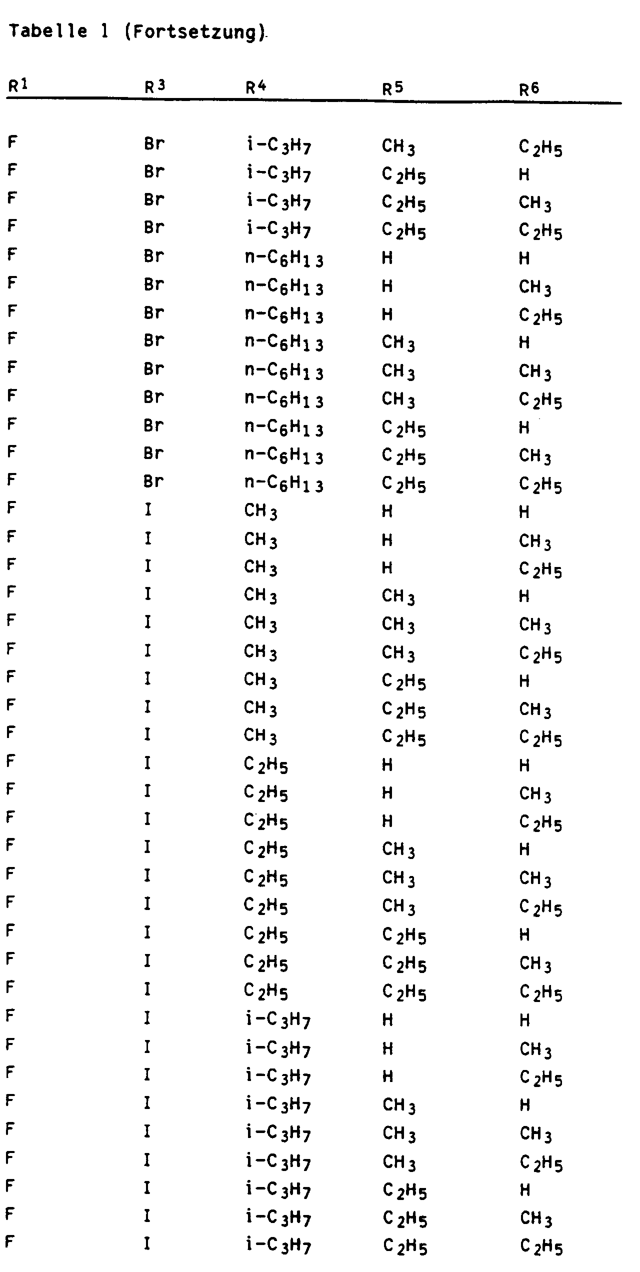

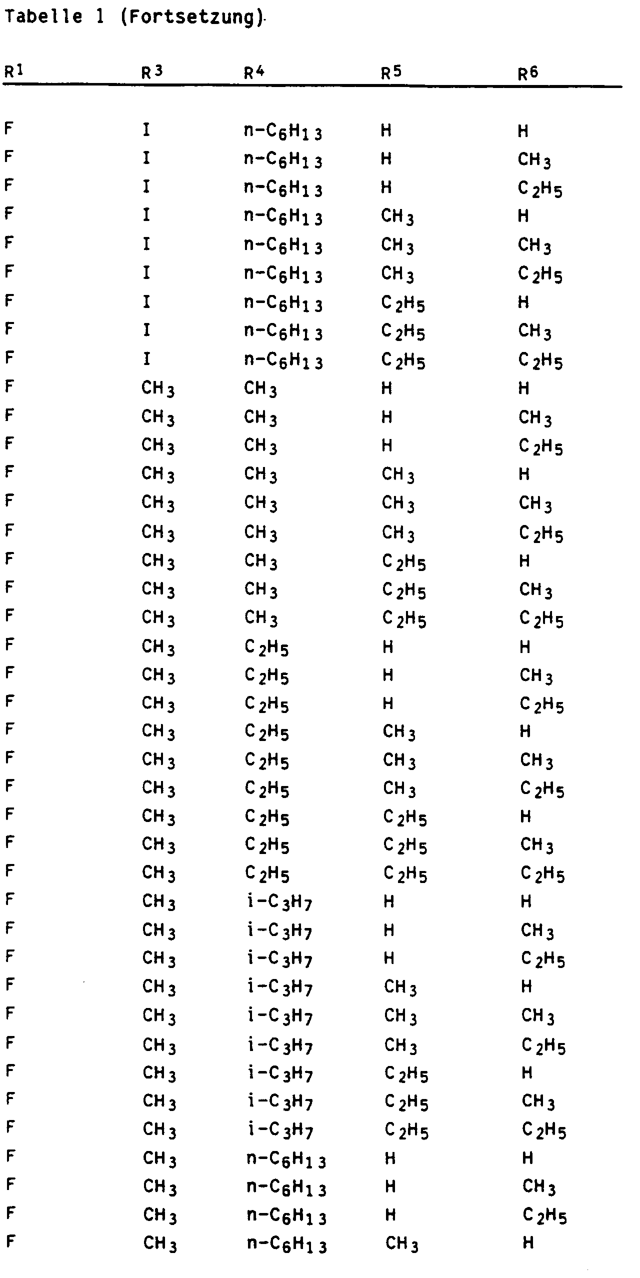

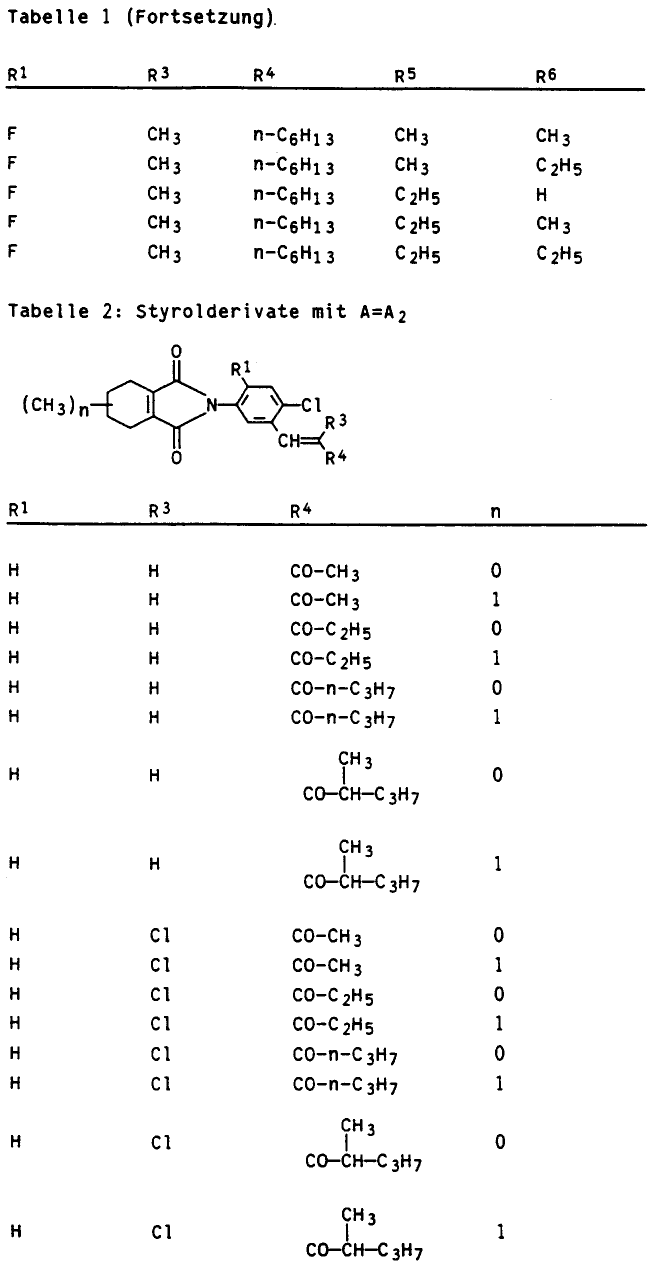

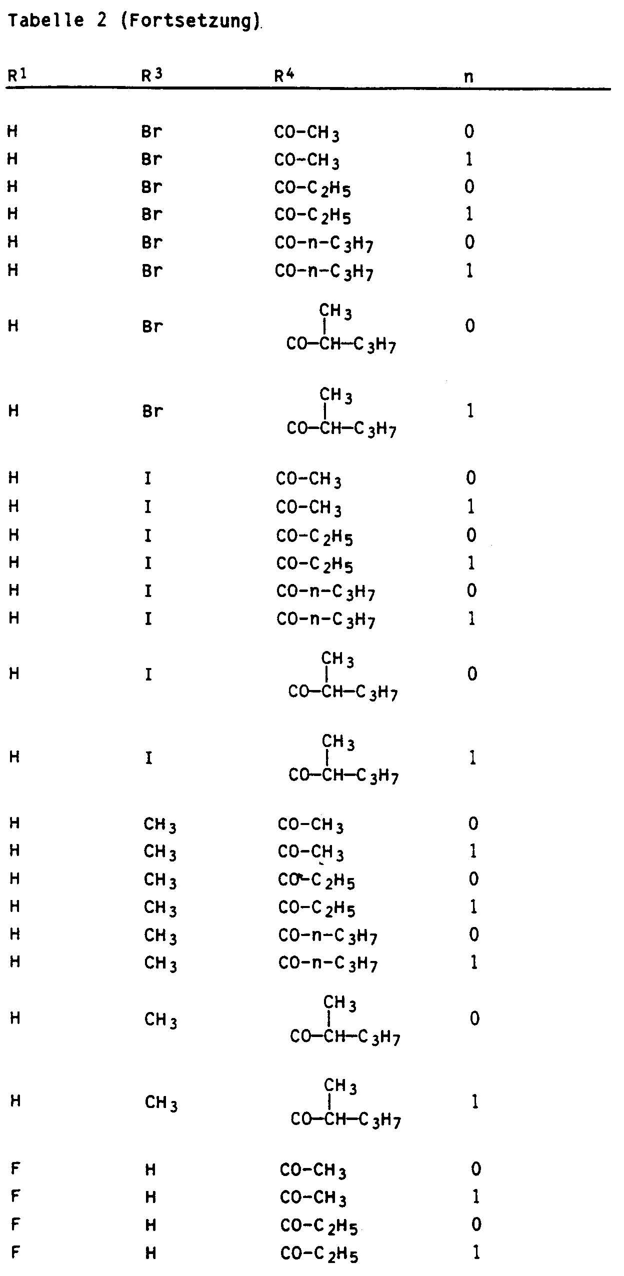

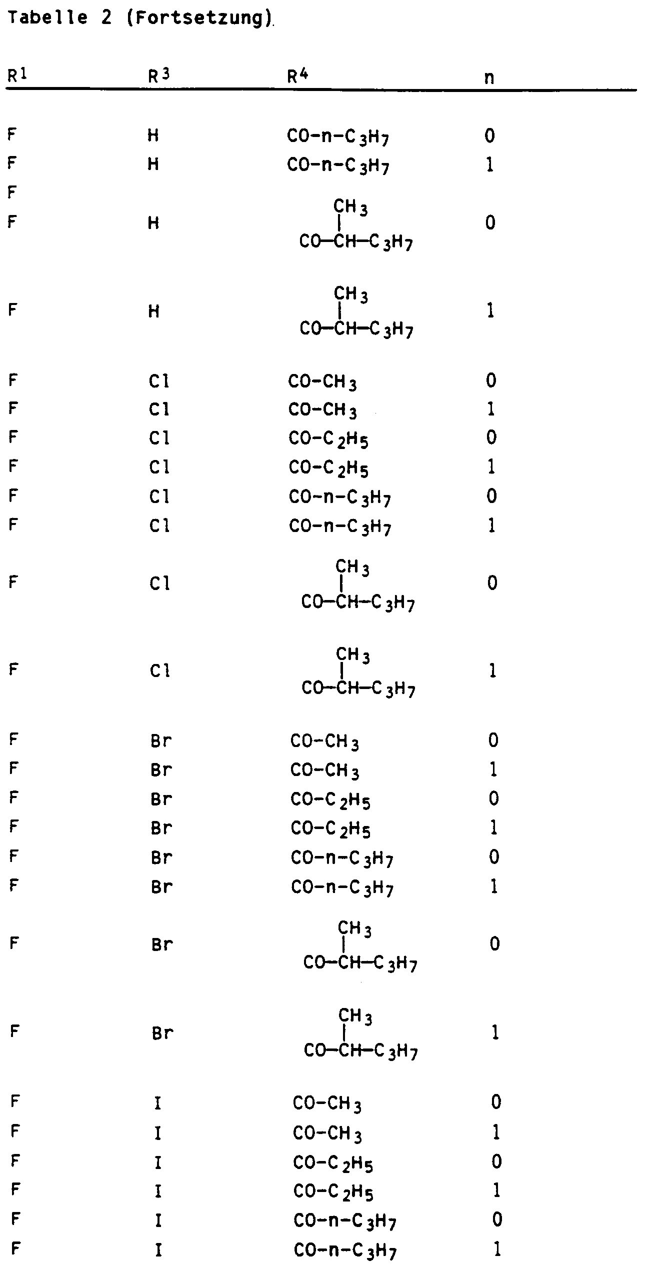

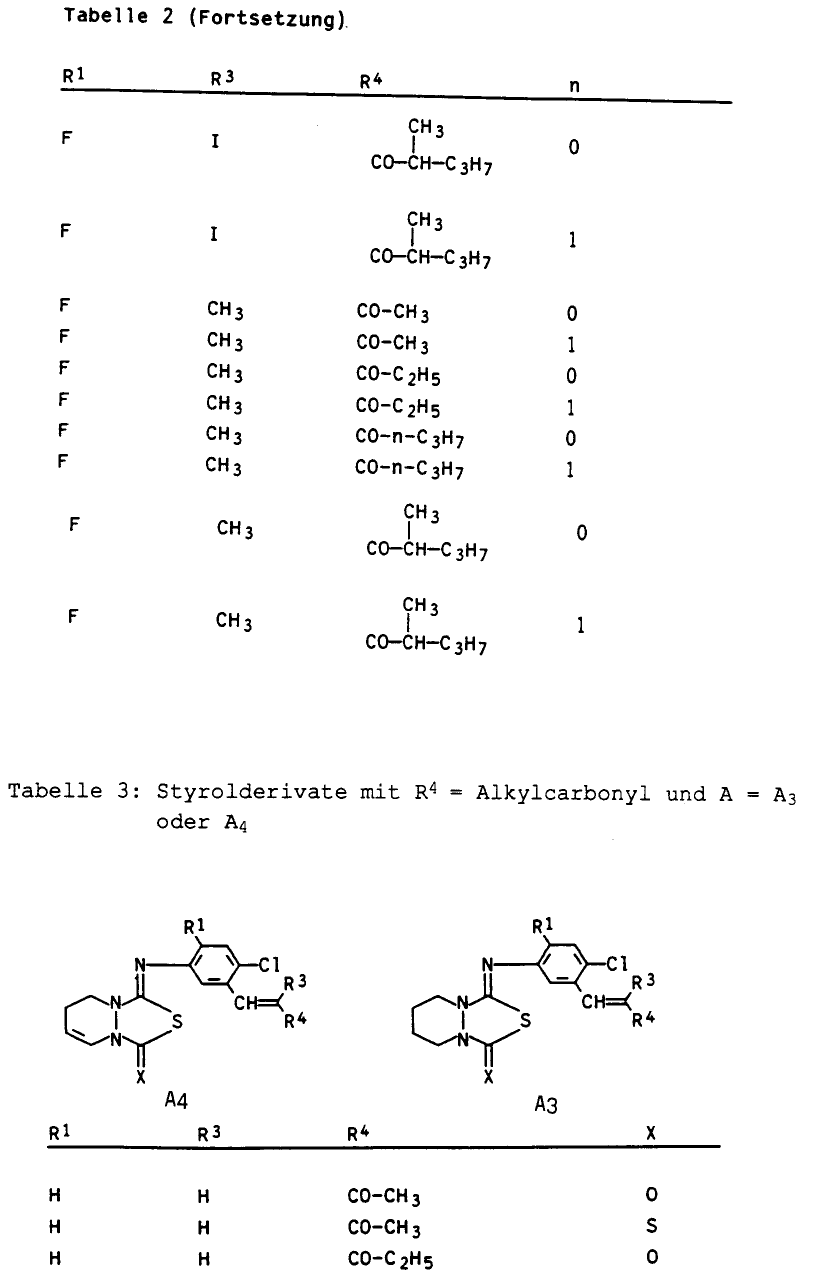

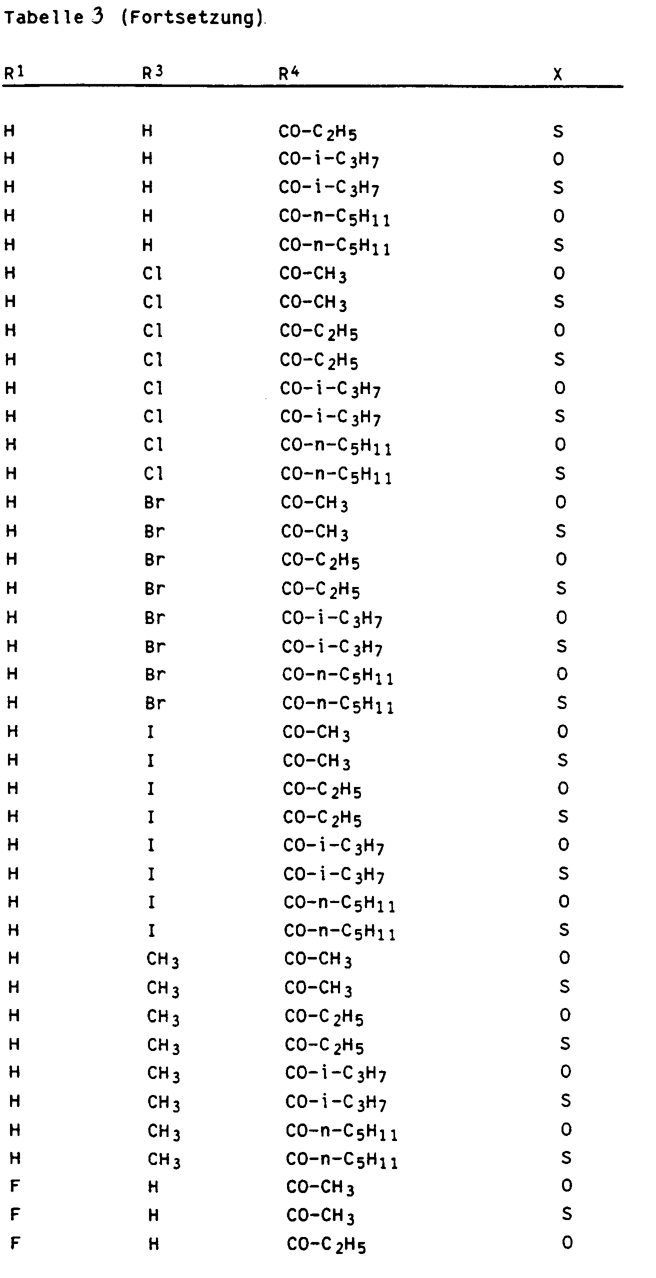

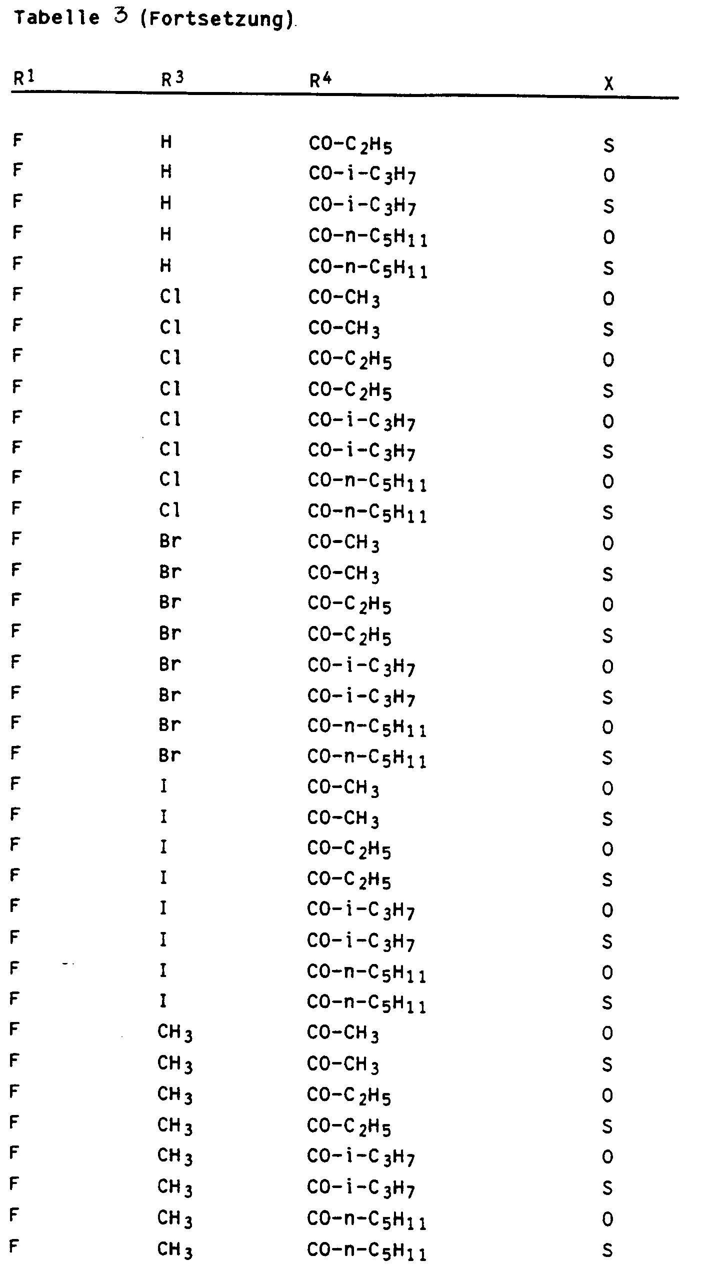

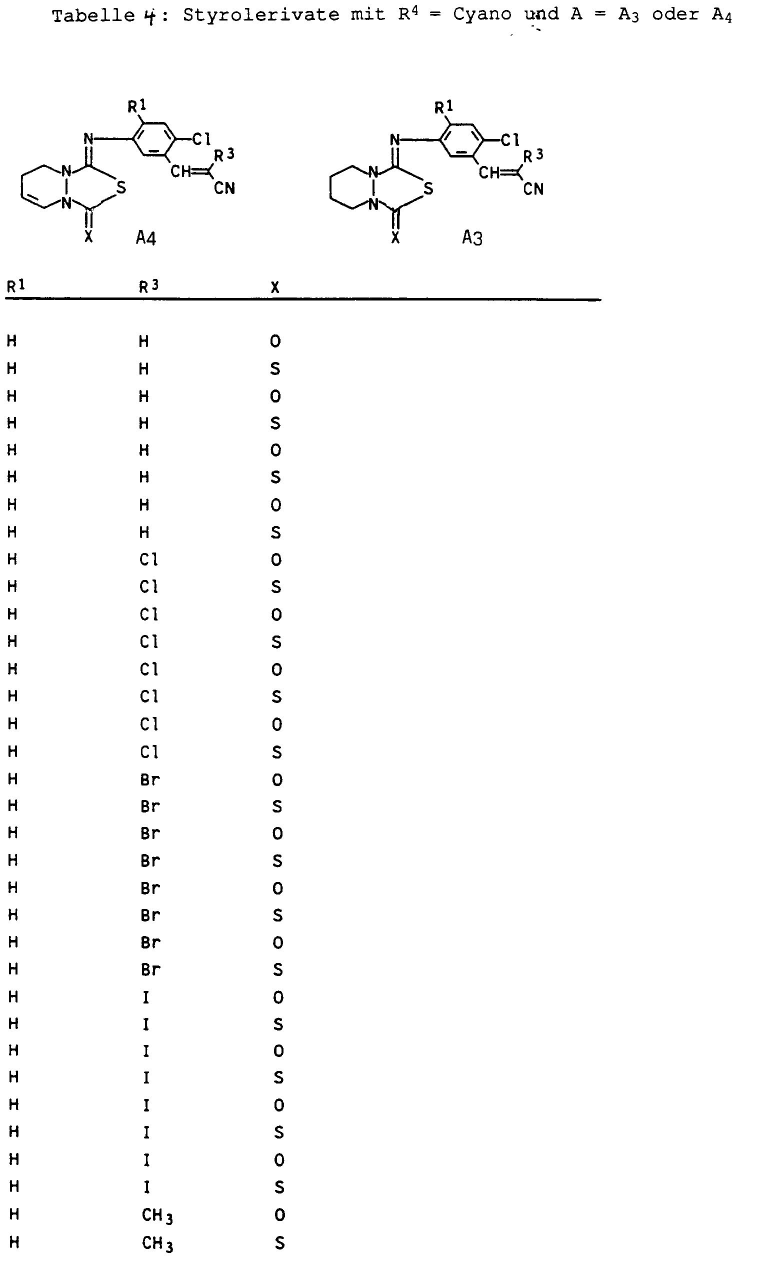

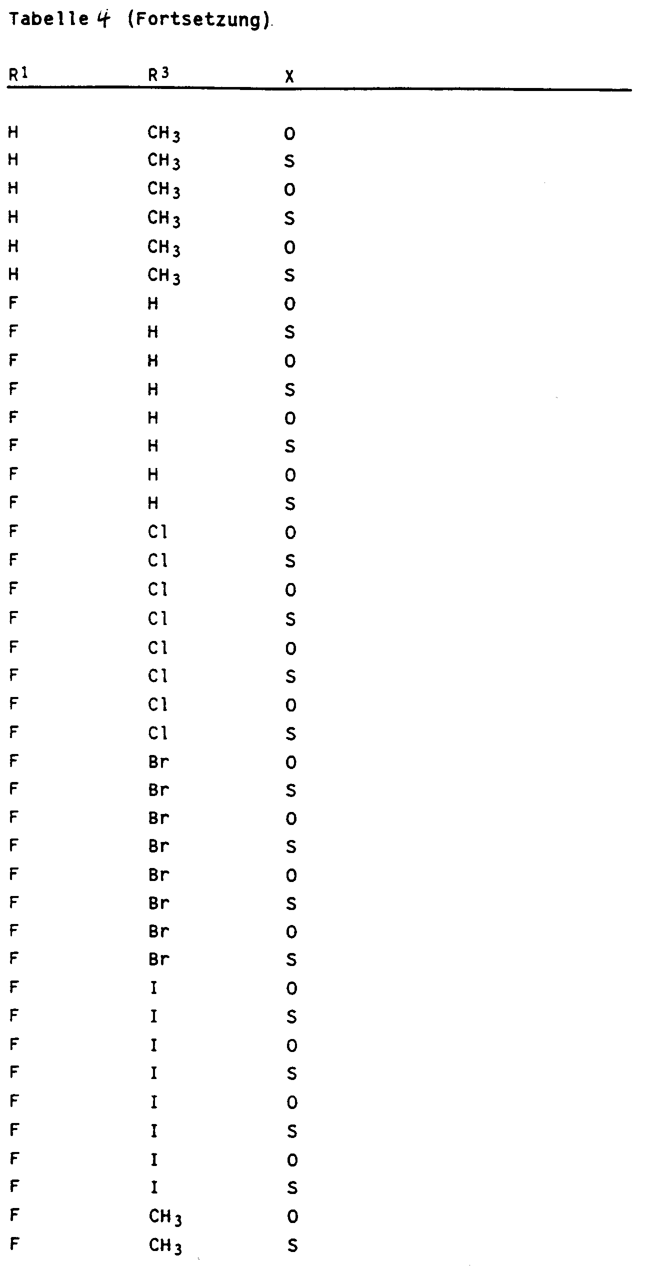

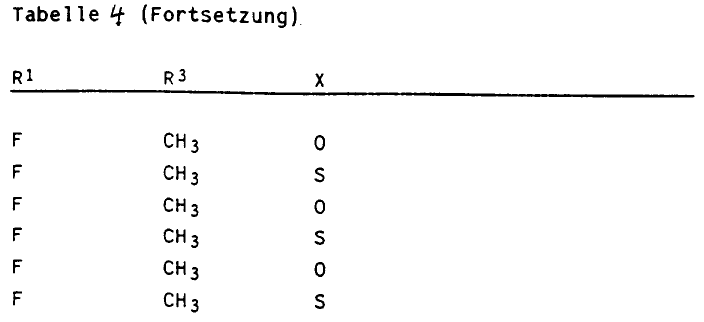

Die Styrolderivate I eignen sich, sowohl als Isomerengemische als auch in Form der reinen Isomeren, als Herbizide. Die Verbindungen I bzw. die sie enthaltenden herbiziden und entblätternden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Styrolderivate I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie . Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. Man vermischt 90 Gewichtsteile der Verbindung Nr. 2 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs Nr. 4 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
IV. 3 Gewichtsteile des Wirkstoffs Nr. 6 werden mit 97 Gewichtsteilen feinteiligem Kaolin vermischt. Man erhält auf diese Weise ein Stäubemittel, das 3 Gew.% des Wirkstoffs enthält.
V. 30 Gewichtsteile des Wirkstoffs Nr. 7 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VI. 20 Gewichtsteile des Wirkstoffs Nr. 9 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3, vorzugsweise 0,01 bis 1 kg/ha aktive Substanz (a.S.).

Die Verbindungen I können daneben zur Dessikation von Baumwolle eingesetzt werden, da sie die Ausbildung von Trenngewebe zwischen Blatt- und Sproßteil der Pflanze fördern.

Außerdem führt die Verkürzung des Zeitintervalls, in dem die einzelnen Baumwollpflanzen reif werden, zu einer erhöhten Faserqualität nach der Ernte.

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

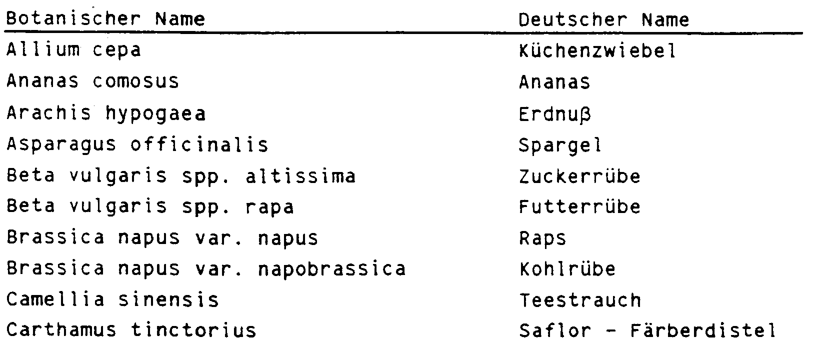

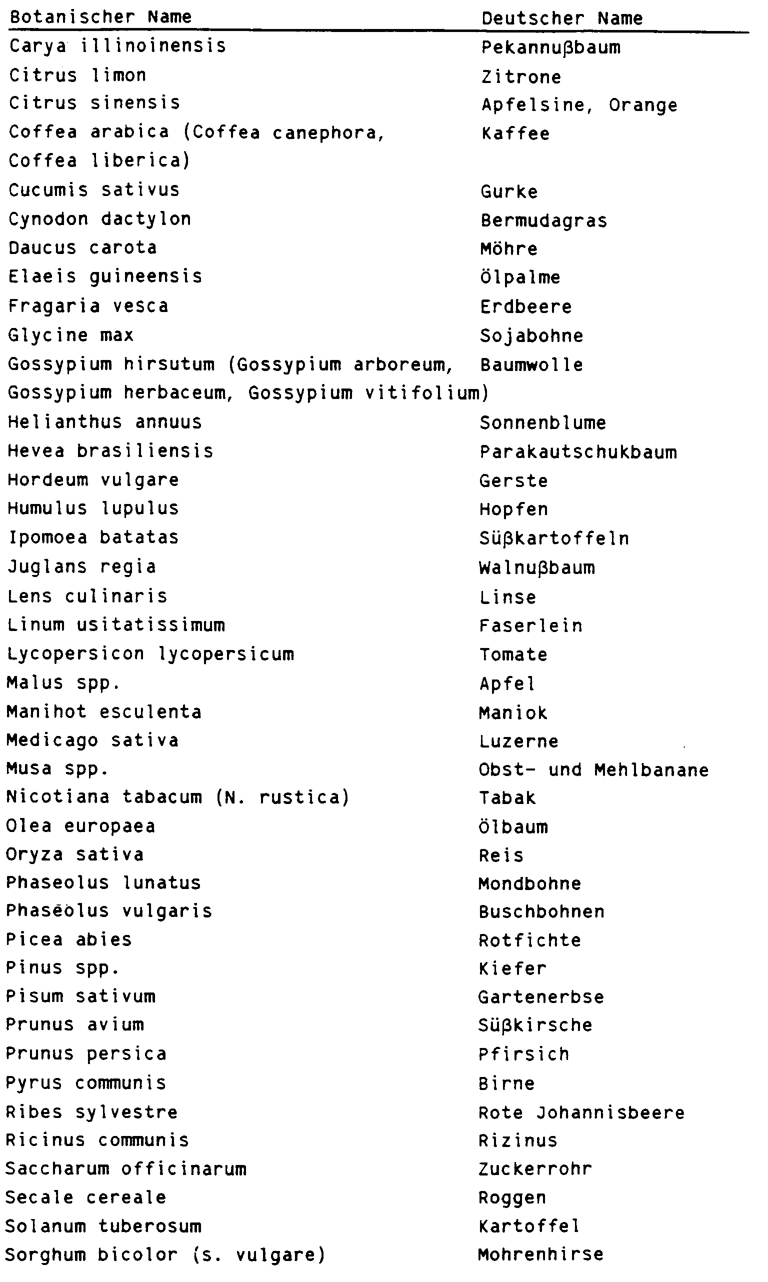

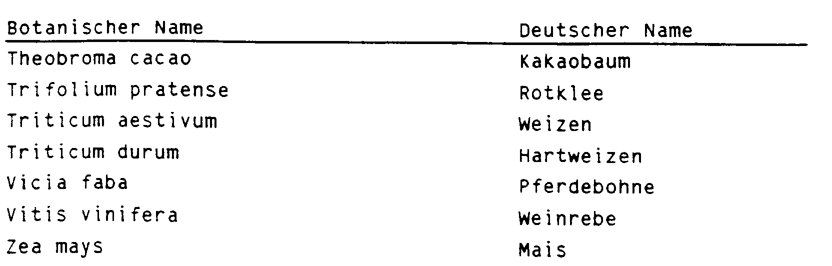

Zur Verbreiterung des Wirkungsspektrums und zur Erzielung synergistischer Effekte können die Styrolderivate I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffe, Aryloxy-, Heteroaryloxyphenoxypropionsäuren sowie deren Salze, Ester und Amide und andere in Betracht.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### N-(3-(2-Chlorbut-1-en-3-onyl)-4-chlorphenyl)-dimethylmaleinsäureimid (Verbindung Nr. 1)

70 ml einer Lösung aus 12,3 g (35 mmol) Acetyl-(chlor)-methylentriphenylphosphoran in Ethanol wurden bei Raumtemperatur vorgelegt und 2,1 g (8 mmol) N-(4-Chlor-3-formylphenyl)-dimethylmaleinsäureimid zugegeben. Nach 4 Stunden Rühren bei ca. 20 bis 25°C kühlte man den Ansatz auf (-10)°C, wonach der Feststoffanteil abgetrennt, mit wenig kaltem Ethanol gewaschen und unter reduziertem Druck bei 40°C getrocknet wurde. Ausbeute: 89 %; Fp.: 173-179°C.

### Beispiel 2

### N-(5-(2-Chlorbut-1-en-3-onyl)-4-chlor-2-fluorphenyl)-4,5,6,7-tetrahydrophthalsäureimid (Verbindung Nr. 2)

12,3 g (35 mmol) Acetyl-(chlor)-methylentriphenylphosphoran wurden in 70 ml Ethanol vorgelegt und 2,15 g (7 mmol) N-(4-Chlor-2-fluor-5-formylphenyl)-4,5,6,7-tetrahydrophthalsäureimid zugegeben. Man rührte 1 Stunde bei etwa 20°C, kühlte auf (-10)°C ab, saugte ab und wusch mit wenig kaltem Ethanol nach. Durch Einengen der Mutterlauge konnte weiterer Feststoff isoliert werden. Das Rohprodukt wurde unter reduziertem Druck bei 40°C getrocknet. Ausbeute: 67 %; Fp.: 141-142,5°C.

### Beispiel 3

### N-(5-(2-Chlorbut-1-en-3-onyl)-2,4-diffluorphenyl)-4,5,6,7-tetranydrophthalsäureimid (Verbindung Nr. 3)

12,3 g (35 mmol) Acetyl-(chlor)-methylentriphenylphosphoran wurden in 70 ml Ethanol vorgelegt und 2,3 g (8 mmol) N-(2,4-Difluor-5-formylphenyl)-4,5,6,7-tetrahydrophthalsäureimid zugegeben. Man rührte 1 Stunde bei 20 bis 25°C, kühlte auf (-10)°C ab, saugte ab und wusch mit wenig kaltem Ethanol nach. Der Rückstand wurde bei 35°C unter reduziertem Druck getrocknet. Ausbeute: 82 %; Fp.: 170-172°C.

Analog den Beispielen 2 bis 4 wurden unter entsprechender Abwandlung der Ausgangsverbindungen die in Tabelle 5 aufgelisteten Styrolderivate I hergestellt:

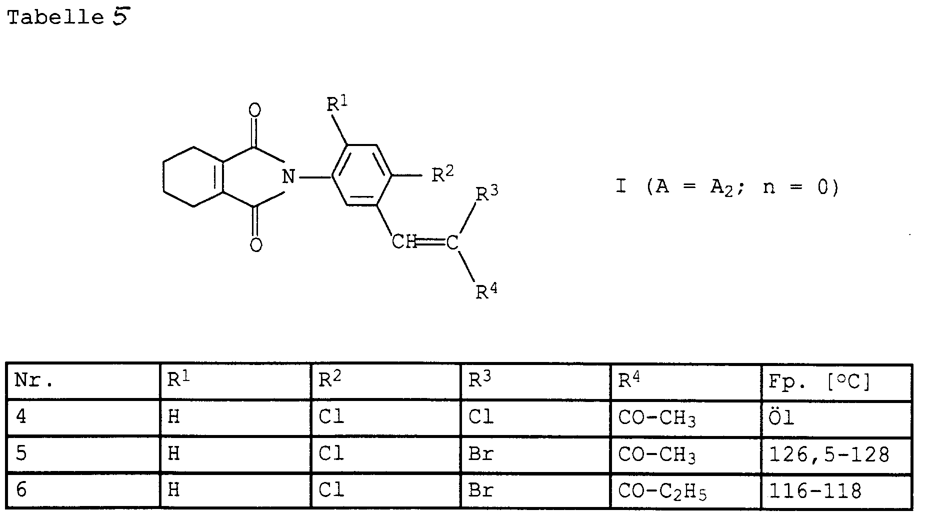

### Beispiel 4

### N-(3-(2-Chlor-2-cyanoethenyl)-4-chlorphenyl)-dimethylmaleinsäureimid (Verbindung Nr. 7)

Zu einer Mischung aus 8,4 g (25 mmol) Chlor-(cyano)-methylentriphenylphosphoran in 40 ml Methanol wurden 2,6 g (10 mmol) N-(4-Chlor-3-formylphenyl)-dimethylmaleinsäureimid gegeben. Man rührte 4 Stunden bei etwa 20 bis 25°C und kühlte dann auf (-10)°C ab, wonach der Feststoffanteil abgetrennt und mit wenig kaltem Methanol nachgewaschen wurde. Der Rückstand wurde unter reduziertem Druck bei 30°C getrocknet. Ausbeute: 69 %; Fp.: 139-140,5°C.

Analog zu Beispiel 4 wurden die Verbindungen der Tabelle 6 hergestellt:

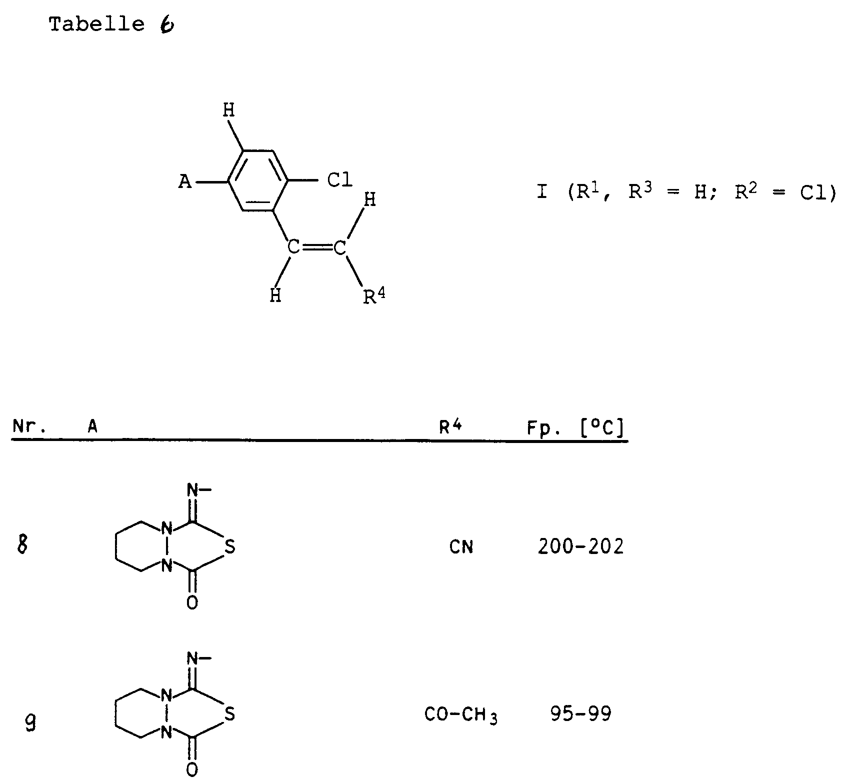

### Anwendungsbeispiele

Die herbizide Wirkung der Styrolderivate I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchs form erst bei einer Wuchshöhe von 3 bis 15 cm mit den in Wasser suspendierten oder emulgierten Wirkstoffen behandelt. Die Aufwandmenge für die Nachauflaufbehandlung betrug 0,03 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Amaranthus retroflexus | Zurückgekrümmter Fuchsschwanz |
| Oryza sativa | Reis |
| Solanum nigrum | Schwarzer Nachtschatten |
| Ipomoea subspecies | Prunkwindearten |
| Triticum aestivum | Sommerweizen |

Mit 0,03 und 0,015 kg/ha a.S. im Nachauflaufverfahren eingesetzt, lassen sich mit dem Beispiel 2 breitblättrige unerwünschte Pflanzen sehr gut bekämpfen. Gleichzeitig ist die Verbindung 2 verträglich für die Kulturpflanzen Sommerweizen und Reis.

## Patentansprüche (Patentansprüche für folgende(n) Vertragsstaat(en): AT, BE, CH, DE, FR, GB, IT, LI, NL, SE)

1. Styrolderivate der allgemeinen Formel I, 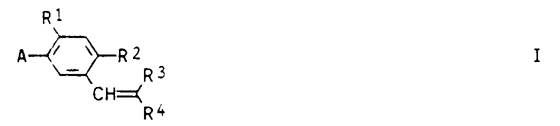 in der die Substituenten folgende Bedeutung haben:
R¹ Wasserstoff oder Halogen;
R² Halogen;
R³ Wasserstoff, Halogen oder C₁-C₄-Alkyl;
R⁴ Cyano oder C₁-C₆-Alkylcarbonyl;
A einenheterocyclischen Rest, ausgewählt aus der Gruppe A₁ bis A₄ 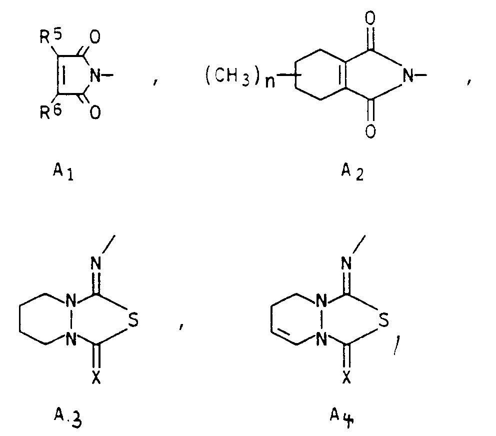 wobei R⁵ und R⁶ Wasserstoff, Methyl oder Ethyl bedeuten, n für 0 oder 1 und X für Sauerstoff oder Schwefel stehen;
mit der Maßgabe, daß R² Chlor und R⁴ C₁-C₆-Alkylcarbonyl bedeuten, wenn A für den Rest A₂ steht;
sowie deren landwirtschaftlich brauchbaren Salze.

2. Verfahren zur Herstellung der Styrolderivate der Formel I gemäß Anspruch 1, dadurch gekennzeichnet, daß man ein Benzaldehydderivat der Formel II 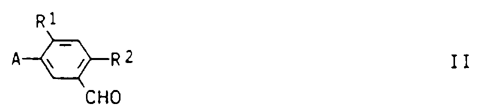 mit einem Phosphoran der Formel III 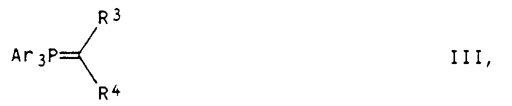 in der Ar für einen Arylrest steht, umsetzt.

3. Herbizide Mittel, enthaltend übliche inerte Zusatzstoffe und mindestens ein Styrolderivat der Formel I gemäß Anspruch 1.

4. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Styrolderivats der Formel I gemäß Anspruch 1 behandelt.

5. Verfahren zur Entblätterung von Baumwolle, dadurch gekennzeichnet, daß man die Baumwollpflanzen mit einer wirksamen Menge eines Styrolderivats der Formel I gemäß Anspruch 1 behandelt.

6. Verwendung der Styrolderivate der Formel I gemäß Anspruch 1 als Herbizide oder Bioregulatoren.

## Patentansprüche (Patentansprüche für folgende(n) Vertragsstaat(en): ES)

1. Herbizides Mittel, enthaltend übliche inerte Zusatzstoffe und mindestens ein Styrolderivat der allgemeinen Formel I 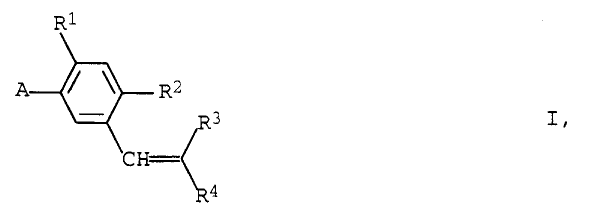 in der die Substituenten folgende Bedeutung haben:
R¹ Wasserstoff oder Halogen;
R² Halogen;
R³ Wasserstoff, Halogen oder C₁-C₄-Alkyl;
R⁴ Cyano oder C₁-C₆-Alkylcarbonyl;
A einen heterocyclischen Rest, ausgewählt aus der Gruppe A₁ bis A₄ 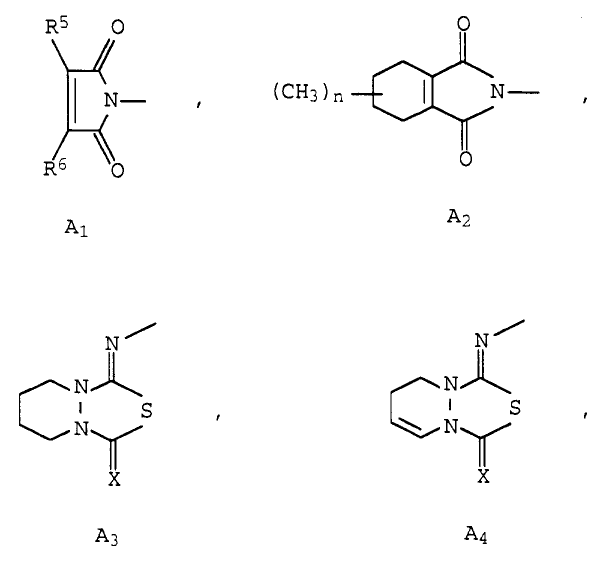 wobei R⁵ und R⁶ Wasserstoff, Methyl oder Ethyl bedeuten, n für 0 oder 1 und X für Sauerstoff oder Schwefel stehen;
mit der Maßgabe, daß R² Chlor und R⁴ C₁-C₆-Alkylcarbonyi bedeuten, wenn A für den Rest A₂ steht;
oder ein landwirtschaftlich brauchbares Salz von I.

2. Verfahren zur Herstellung der Styrolderivate der Formel I gemäß Anspruch 1, dadurch gekennzeichnet, daß man ein Benzaldehydderivat der Formel II 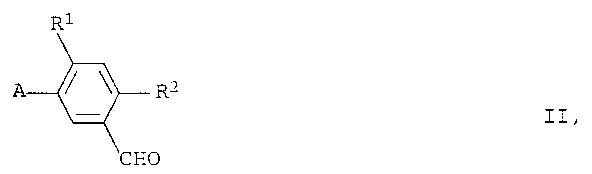 mit einem Phosphoran der Formel III 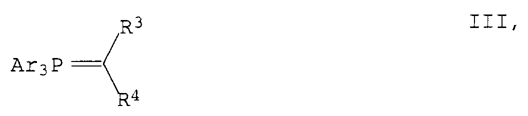 in der Ar für einen Arylrest steht, umsetzt.

3. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Styrolderivats der Formel I gemäß Anspruch 1 behandelt.

4. Verfahren zur Entblätterung von Baumwolle, dadurch gekennzeichnet, daß man die Baumwollpflanzen mit einer wirksamen Menge eines Styrolderivats der Formel I gemäß Anspruch 1 behandelt.

5. Verwendung der Styrolderivate der Formel I gemäß Anspruch 1 als Herbizide oder Bioregulatoren.

## Claims (Claims for the following Contracting State(s): AT, BE, CH, DE, FR, GB, IT, LI, NL, SE)

1. A styrene derivative of the formula I 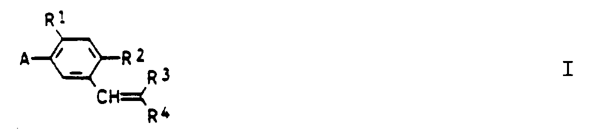 where R¹ is hydrogen or halogen, R² is halogen, R³ is hydrogen, halogen or C₁-C₄-alkyl, R⁴ is cyano or C₁-C₆-alkylcarbonyl and A is a heterocyclic radical selected from the groups A₁ to A₄ 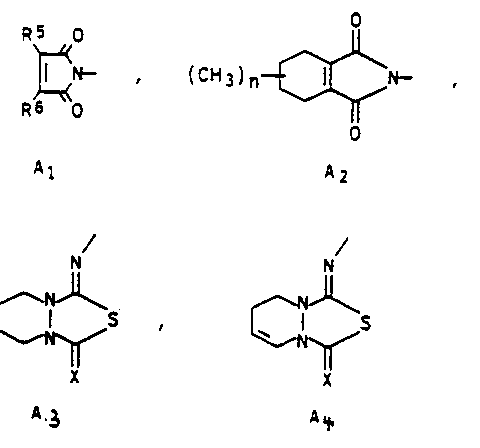 where R⁵ and R⁶ are each hydrogen, methyl or ethyl, n is 0 or 1 and X is oxygen or sulfur, with the proviso that R² is chlorine and R⁴ is C₁-C₆-alkylcarbonyl when A is A₂, and agriculturally useful salts thereof.

2. A process for the preparation of a styrene derivative of the formula I as claimed in claim 1, wherein a benzaldehyde derivative of the formula II 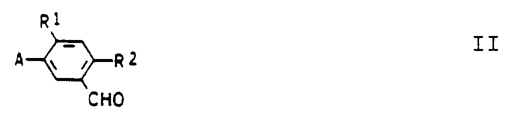 is reacted with a phosphorane of the formula III 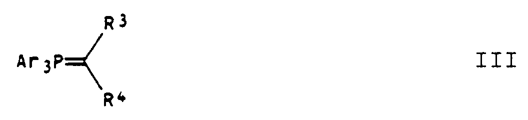 where Ar is aryl.

3. A herbicide containing conventional inert additives and at least one styrene derivative of the formula I as claimed in claim 1.

4. A method for controlling undesirable plant growth, wherein the undesirable plants and/or their habitat are or is treated with a herbicidal amount of a styrene derivative of the formula I as claimed in claim 1.

5. A method for the defoliation of cotton, wherein the cotton plants are treated with an effective amount of a styrene derivative of the formula I as claimed in claim 1.

6. Use of a styrene derivative of the formula I as claimed in claim 1 as a herbicide or bioregulator.

## Claims (Claims for the following Contracting State(s): ES)

1. A herbicide containing conventional inert additives and at least one styrene derivative of the formula I 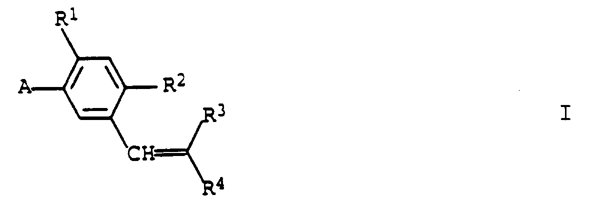 where R¹ is hydrogen or halogen, R² is halogen, R³ is hydrogen, halogen or C₁-C₄-alkyl, R⁴ is cyano or C₁-C₆-alkylcarbonyl and A is a heterocyclic radical selected from the groups A₁ to A₄ 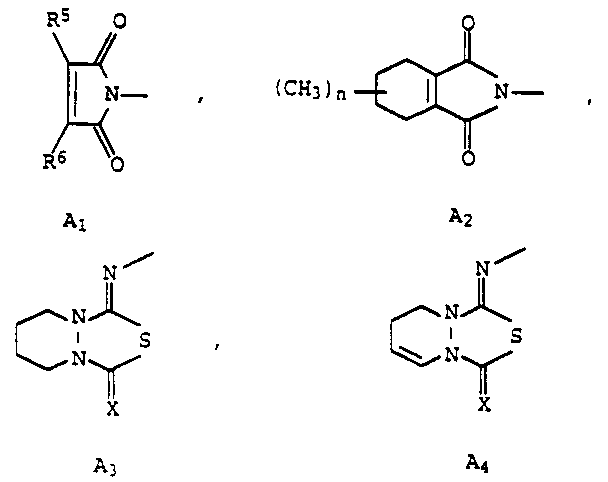 where R⁵ and R⁶ are each hydrogen, methyl or ethyl, n is 0 or 1 and X is oxygen or sulfur, with the proviso that R² is chlorine and R⁴ is C₁-C₆-alkylcarbonyl when A is A₂, or an agriculturally useful salt of I.

2. A process for the preparation of a styrene derivative of the formula I as claimed in claim 1, wherein a benzaldehyde derivative of the formula II 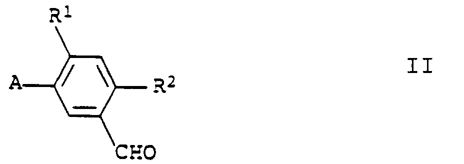 is reacted with a phosphorane of the formula III 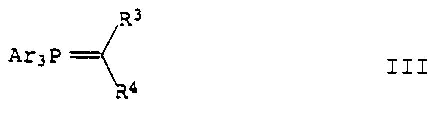 where Ar is aryl.

3. A method for controlling undesirable plant growth, wherein the undesirable plants and/or their habitat are or is treated with a herbicidal amount of a styrene derivative of the formula I as claimed in claim 1.

4. A method for the defoliation of cotton, wherein the cotton plants are treated with an effective amount of a styrene derivative of the formula I as claimed in claim 1.

5. Use of a styrene derivative of the formula I as claimed in claim 1 as a herbicide or bioregulator.

## Revendications (Revendications pour l'(les) Etat(s) contractant(s) suivant(s): AT, BE, CH, DE, FR, GB, IT, LI, NL, SE)

1. Dérivés du styrène répondant à la formule générale I 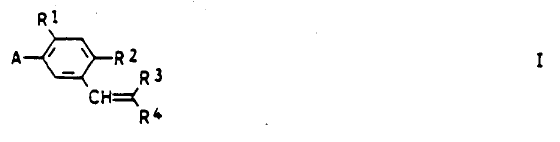 dans laquelle les symboles ont les significations suivantes :
R¹ représente l'hydrogène ou un halogène ;
R² représente un halogène ;
R³ représente l'hydrogène, un halogène ou un groupe alkyle en C1-C4 :
R⁴ représente un groupe cyano ou (alkyle en C1-C6)carbonyle ;
A représente un radical hétérocyclique choisi parmi les groupes A₁ à A₄ 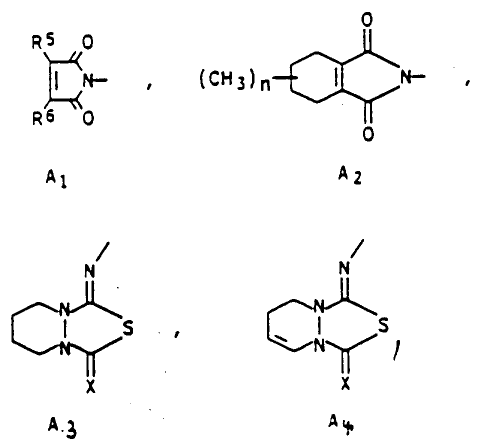 dans lesquels R⁵ et R⁶ représentent l'hydrogène, des groupes méthyle ou éthyle, n est égal à 0 ou 1 et X représente l'oxygène ou le soufre ;
sous réserve que R² doit représenter le chlore et R⁴ un groupe (alkyle en C1-C6)carbonyle lorsque A consiste en le groupe A₂ ;
et leurs sels aptes à des applications agricoles.

2. Procédé de préparation des dérivés du styrène répondant à la formule I de la revendication 1, caractérisé par le fait que l'on fait réagir un dérivé du benzaldéhyde répondant à la formule II 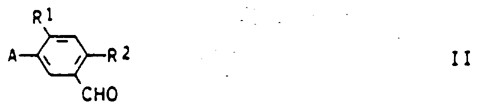 avec un phosphorane de formule III 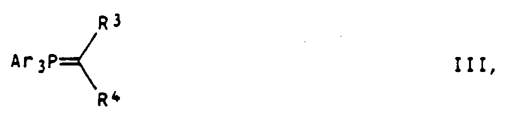 dans laquelle Ar représente un groupe aryle.

3. Produit herbicide contenant des additifs inertes usuels et au moins un dérivé du styrène de formule I selon la revendication 1.

4. Procédé pour combattre les croissances de végétaux indésirables, caractérisé par le fait que l'on traite les végétaux indésirables et/ou leur biotope par une quantité herbicide efficace d'un dérivé du styrène de formule I selon la revendication 1.

5. Procédé pour la défoliation du coton, caractérisé par le fait que l'on traite les plants de coton par une quantité efficace d'un dérivé du styrène de formule I selon la revendication 1.

6. Utilisation des dérivés du styrène de formule I selon la revendication 1, en tant qu'herbicides ou biorégulateurs.

## Revendications (Revendications pour l'(les) Etat(s) contractant(s) suivant(s): ES)

1. Produit herbicide contenant des additifs inertes usuels et au moins un dérivé du styrène répondant à la formule générale I 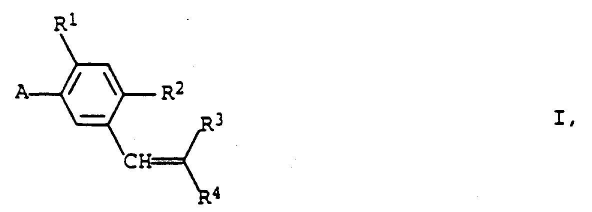 dans laquelle les symboles ont les significations suivantes :
R¹ représente l'hydrogène ou un halogène ;
R² représente un halogène ;
R³ représente l'hydrogène, un halogène ou un groupe alkyle en C1-C4 ;
R⁴ représente un groupe cyano ou (alkyle en C1-C6)carbonyle ;
A représente un groupe hétérocyclique choisi parmi les groupes A₁ à A₄ 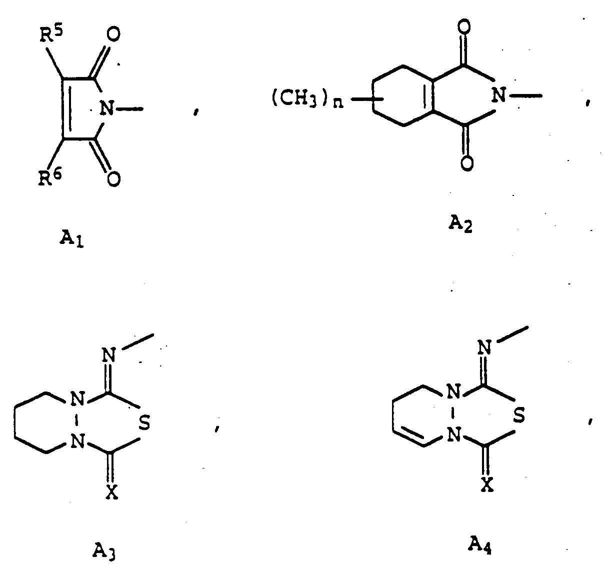 dans lesquels R⁵ et R⁶ représentent l'hydrogène, des groupes méthyle ou éthyle, n est égal à 0 ou 1 et X représente l'oxygène ou le soufre ;
sous réserve que R² doit représenter le chlore et R⁴ un groupe (alkyle en C1-C6)carbonyle lorsque A consiste en le groupe A₂ ;
ou un sel de I apte aux applications agricoles.

2. Procédé de préparation des dérivés du styrène de formule I selon la revendication 1, caractérisé par le fait que l'on fait réagir un dérivé du benzaldéhyde de formule II 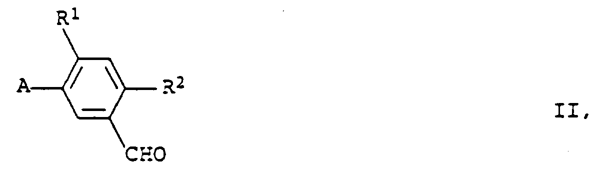 avec un phosphorane de formule III 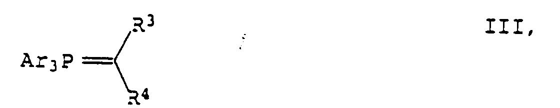 dans laquelle Ar représente un groupe aryle.

3. Procédé pour combattre les croissances de végétaux indésirables, caractérisé par le fait que l'on traite les végétaux indésirables et/ou leur biotope par une quantité herbicide efficace d'un dérivé du styrène de formule I selon la revendication 1.

4. Procédé pour la défoliation du coton, caractérisé par le fait que l'on traite les plants de coton par une quantité efficace d'un dérivé du styrène de formule I selon la revendication 1.

5. Utilisation des dérivés du styrène de formule I selon la revendication 1 en tant qu'herbicides ou biorégulateurs.
